# EUROPEAN PATENT APPLICATION

(11) **EP 4 685 350 A1**
(43) Date of publication of application: **28.01.2026**
(21) Application number: 24811040.5
(22) Date of filing: 16.05.2024
(51) Int. Cl.: F04B 43/02, A61M 1/16

(54) **PUMP DEVICE**

(30) Priority: 19.05.2023 JP 2023083478
(71) Applicant: Nikkiso Company Limited, Tokyo 150-6022 (JP)
(72) Inventor: TOKUNAGA, Makoto, Higashimurayama-shi, Tokyo 189-8520 (JP); MOCHIZUKI, Hiroaki, Higashimurayama-shi, Tokyo 189-8520 (JP)
(74) Representative: von Hirschhausen, Helge
(86) International application number: PCT/JP2024/018243
(87) International publication number: WO 2024/242033

(57) **Abstract**

Provided is a pump device in which remaining dialysate or drain liquid is prevented from scattering to the outside when a chamber is detached from a pump body. A pump device 1 includes a supply chamber 2 having a supply-side accommodation part S1; a drain chamber 3 having a drain-side accommodation part S2; dividing membranes 4 each being a flexible member that closes a corresponding one of the supply-side accommodation part S1 and the drain-side accommodation part S2, the dividing membranes 4 being displaceable relative to the supply-side accommodation part S1 and the drain-side accommodation part S2 in such a manner as to cause dialysate to be suctioned into and discharged from the supply-side accommodation part S1 through a liquid-supply-side connection port and cause drain liquid to be suctioned into and discharged from the drain-side accommodation part S2 through a liquid-drain-side connection port; and a pump body 7 from and to which the supply chamber 2 or the drain chamber 3 is detachable and attachable with the supply-side accommodation part S1 or the drain-side accommodation part S2 being closed by the corresponding dividing membrane 4, the pump body 7 including a piston P configured to move with activation of a motor M and in such a manner as to displace the dividing membranes 4.

## Description

### Technical Field

The present invention relates to a pump device configured to suction and discharge dialysate into and from a supply-side accommodation part through a liquid-supply-side connection port and to suction and discharge drain liquid into and from a drain-side accommodation part through a liquid-drain-side connection port.

### Background Art

A blood purifier, such as a dialyzer, intended for hemodialysis treatment is provided with a dialysate supply line for supplying dialysate and a dialysate drain line for discharging drain dialysate. The dialysate supply line and the dialysate drain line extend from a dialysis device and are connected to the blood purifier, thereby enabling the dialysate to be supplied to the blood purifier and simultaneously enabling the drain dialysate to be discharged from the blood purifier.

The dialysis device includes a pump device provided astride the dialysate supply line and the dialysate drain line. One existing pump device (first existing example) is a reciprocating metering pump including a cylinder constituted by a pair of pump chambers provided with respective pistons, as disclosed in PTL 1 for example. In such a reciprocating metering pump, an electric motor or the like activates a crank mechanism to cause the pistons to undergo a reciprocating motion inside the cylinders, whereby the delivery of dialysate to a dialyzer is enabled.

Another existing pump device (second existing example) includes, as disclosed in PTL 2 for example, a chamber sectioned by an elastic member, such as a diaphragm, into a liquid-supply-side area and a liquid-drain-side area; and electromagnetic valves provided to respective flow paths connected to the liquid-supply-side area and the liquid-drain-side area. In such a pump device, the electromagnetic valves are controlled to open and close the respective flow paths with arbitrary timings, whereby the delivery of dialysate to a dialyzer is enabled.

### Citation List

### Patent Literature

PTL 1: Japanese Unexamined Patent Application Publication No. S55-167009
PTL 2: Japanese Unexamined Patent Application Publication No. S53-142382

### Summary of Invention

### Technical Problem

In the pump device according to the first existing example, however, liquid leakage may occur at sealed parts while the pistons are undergoing the reciprocating motion inside the cylinder. In the pump device according to the second existing example, the plurality of electromagnetic valves need to be controlled with high accuracy, and another pump needs to be provided for liquid delivery to the chamber. In view of the above, the present applicant has reached an examination in which the suction and discharge of dialysate and the suction and discharge of drain liquid are enabled by displacing an elastic member, such as a diaphragm, with a movement of a piston that is activated by a drive source. In such a configuration, however, when the chamber is detached from the pump device for replacement of the chamber, dialysate or drain liquid remaining in the chamber may scatter to the outside.

The present invention has been conceived in view of the above circumstances and provides a pump device in which remaining dialysate or drain liquid is prevented from scattering to the outside when a chamber is detached from a pump body.

### Solution to Problem

A pump device according to an embodiment of the present invention includes a supply chamber to which a liquid-supply-side connection port is connected and that has a supply-side accommodation part configured to accommodate a predetermined volume of dialysate; a drain chamber to which a liquid-drain-side connection port is connected and that has a drain-side accommodation part configured to accommodate a predetermined volume of drain liquid; dividing membranes each being a flexible member attached to a corresponding one of the supply chamber and the drain chamber in such a manner as to close a corresponding one of the supply-side accommodation part and the drain-side accommodation part, the dividing membranes being displaceable relative to the supply-side accommodation part and the drain-side accommodation part in such a manner as to cause the dialysate to be suctioned into and discharged from the supply-side accommodation part through the liquid-supply-side connection port and cause the drain liquid to be suctioned into and discharged from the drain-side accommodation part through the liquid-drain-side connection port; and a pump body from and to which the supply chamber or the drain chamber is detachable and attachable with the supply-side accommodation part or the drain-side accommodation part being closed by the corresponding dividing membrane, the pump body including a driven part configured to move with activation of a drive source and in such a manner as to displace the dividing membranes. Advantageous Effects of Invention

According to the present invention, the dividing membranes are each a flexible member attached to a corresponding one of the supply chamber and the drain chamber in such a manner as to close a corresponding one of the supply-side accommodation part and the drain-side accommodation part. Furthermore, the supply chamber or the drain chamber is detachable from and attachable to the pump body with the supply-side accommodation part or the drain-side accommodation part being closed by the corresponding dividing membrane. Therefore, remaining dialysate or drain liquid is prevented from scattering to the outside when the chamber is detached from the pump body.

### Brief Description of Drawings

[Fig. 1] Fig. 1 is an external perspective view of a pump device according to an embodiment of the present invention.
[Fig. 2] Fig. 2 is another external perspective view of the pump device.
[Fig. 3] Fig. 3 is a four-view drawing of the pump device.
[Fig. 4] Fig. 4 illustrates a section taken along line IV-IV given in Fig. 3.
[Fig. 5] Fig. 5 is an exploded perspective view of major components included in the pump device.
[Fig. 6] Fig. 6 includes perspective views of a supply chamber or a drain chamber included in the pump device and in a state detached from a pump body.
[Fig. 7] Fig. 7 is a perspective view of a drive source and a driven part included in the pump device.
[Fig. 8] Fig. 8 is a schematic diagram illustrating the piping (flow paths for dialysate and drain liquid) of a hemodialysis apparatus to which the pump device is connected.
[Fig. 9] Fig. 9 is an external perspective view of a pump device according to another embodiment of the present invention.
[Fig. 10] Fig. 10 is a longitudinal sectional view of the pump device.
[Fig. 11] Fig. 11 is a longitudinal sectional view of a pump device according to yet another embodiment of the present invention.

### Description of Embodiments

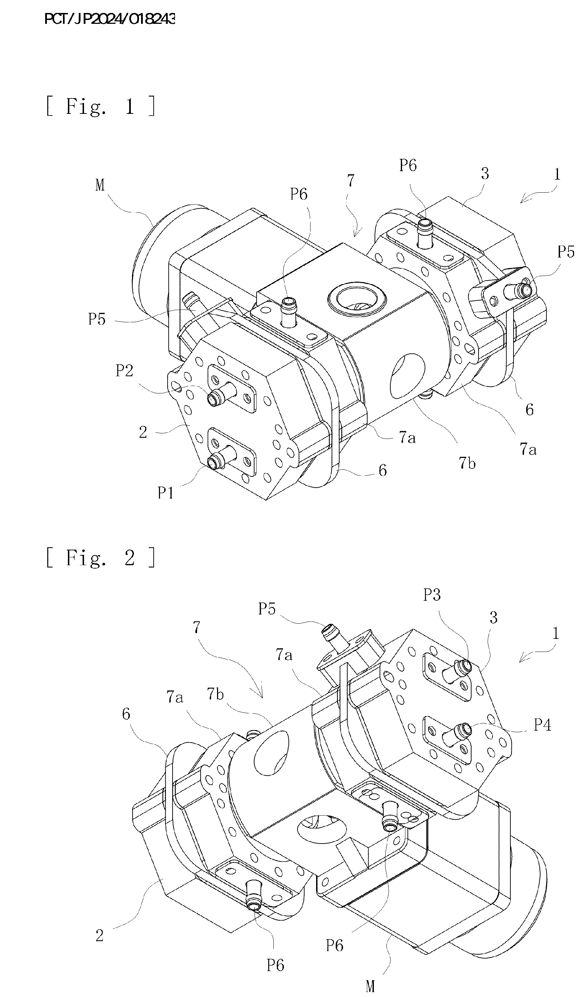

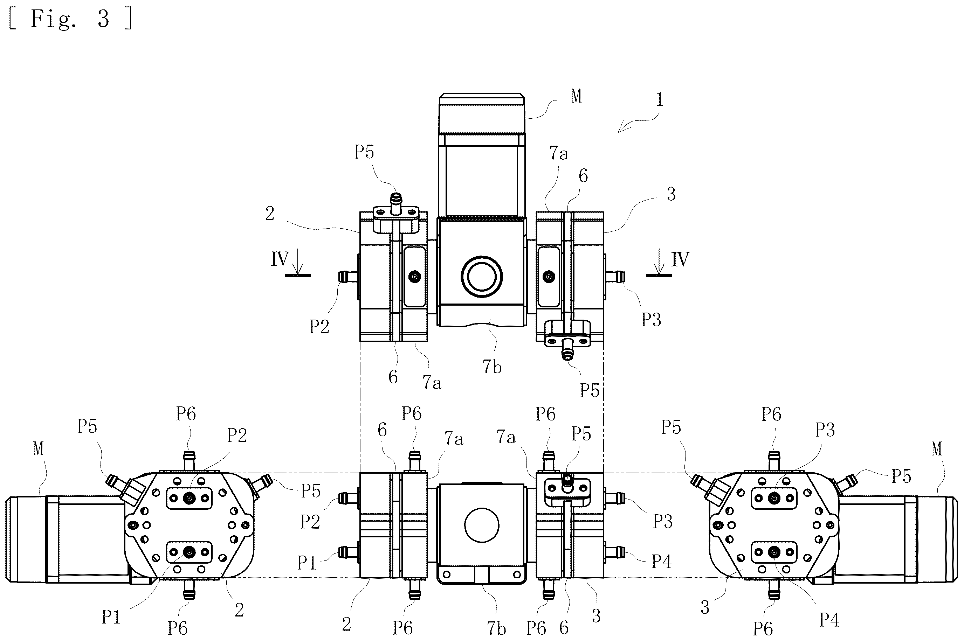

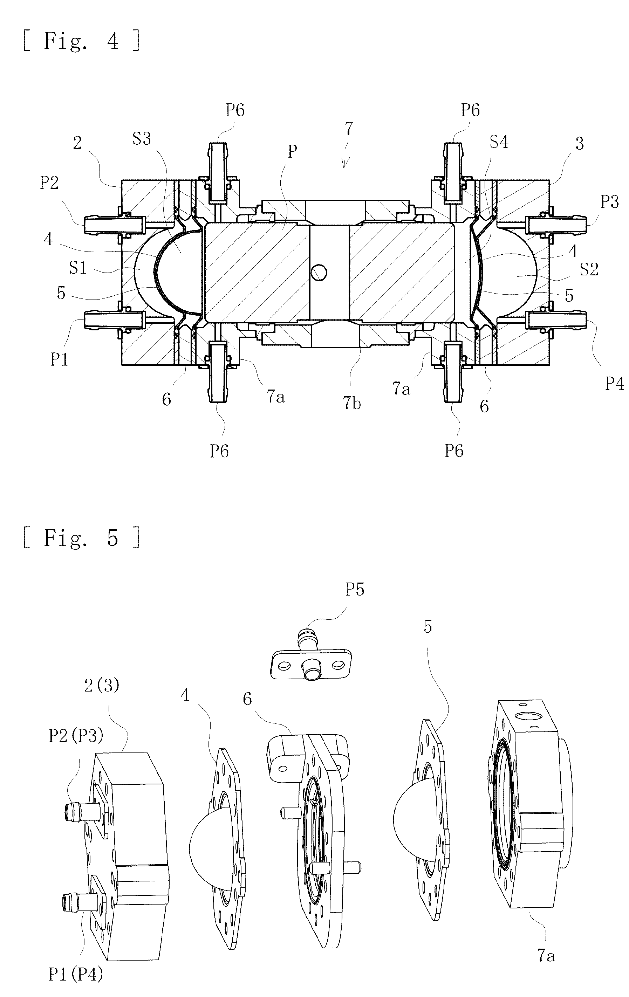

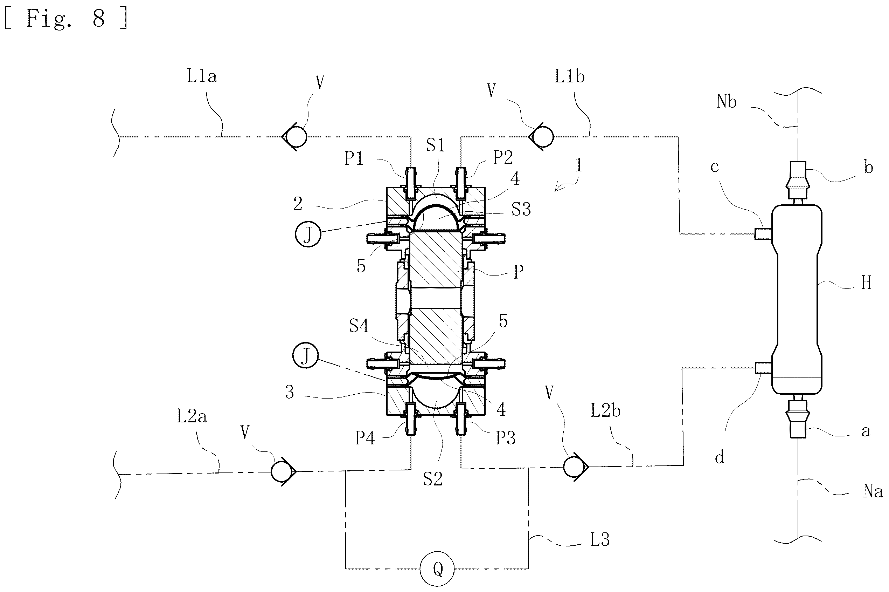

Embodiments of the present invention will now be described specifically with reference to the drawings. A pump device 1 according to the present embodiment is applied to a hemodialysis apparatus as illustrated in Fig. 8 and is configured to supply dialysate to a blood purifier H and simultaneously discharge drain liquid from the blood purifier H. As illustrated in Figs. 1 to 7, the pump device 1 includes a supply chamber 2, a drain chamber 3, dividing membranes 4, moving membranes 5, a pump body 7, and a motor M. The motor M serves as a drive source.

As illustrated in Figs. 1 to 3, the supply chamber 2 is provided at one side of the pump device 1 and is a chamber to which liquid-supply-side connection ports (P1 and P2) are connected and that has a supply-side accommodation part S1, which is configured to accommodate a predetermined volume of dialysate. Specifically, a dividing membrane 4 defines the supply-side accommodation part S1 in the supply chamber 2. With the displacement of the dividing membrane 4, dialysate is suctioned into the supply-side accommodation part S1 through the liquid-supply-side connection port P1, whereas the dialysate is discharged through the liquid-supply-side connection port P2.

The liquid-supply-side connection ports (P1 and P2) are each a connection port projecting from the supply chamber 2. In the present embodiment, as illustrated in Fig. 8, the liquid-supply-side connection port P1 is connected to a dialysate introduction line L1a, which is connected to a dialysate supply source (not illustrated), and the liquid-supply-side connection port P2 is connected to a dialysate introduction line L1b, which is connected to a dialysate introduction port c of the blood purifier H.

As illustrated in Figs. 1 to 3, the drain chamber 3 is provided at the other side of the pump device 1 and is a chamber to which liquid-drain-side connection ports (P3 and P4) are connected and that has a drain-side accommodation part S2, which is configured to accommodate a predetermined volume of drain liquid. Specifically, a dividing membrane 4 defines the drain-side accommodation part S2 in the drain chamber 3. With the displacement of the dividing membrane 4, drain liquid is suctioned into the drain-side accommodation part S2 through the liquid-drain-side connection port P3, whereas the drain liquid is discharged through the liquid-drain-side connection port P4.

The liquid-drain-side connection ports (P3 and P4) are each a connection port projecting from the drain chamber 3. In the present embodiment, as illustrated in Fig. 8, the liquid-drain-side connection port P4 is connected to a dialysate drain line L2a, which is connected to draining means (not illustrated), and the liquid-drain-side connection port P3 is connected to a dialysate drain line L2b, which is connected to a dialysate drain port d of the blood purifier H.

The dividing membranes 4 are each a flexible member (for example, a diaphragm made of a rubber material, a resin material, or the like) attached to a corresponding one of the supply chamber 2 and the drain chamber 3 in such a manner as to close a corresponding one of the supply-side accommodation part S1 and the drain-side accommodation part S2. The dividing membranes 4 are displaceable by bending relative to the supply-side accommodation part S1 and the drain-side accommodation part S2 in such a manner as to cause the dialysate to be suctioned into and discharged from the supply-side accommodation part S1 through the liquid-supply-side connection ports (P1 and P2) and cause the drain liquid to be suctioned into and discharged from the drain-side accommodation part S2 through the liquid-drain-side connection ports (P3 and P4).

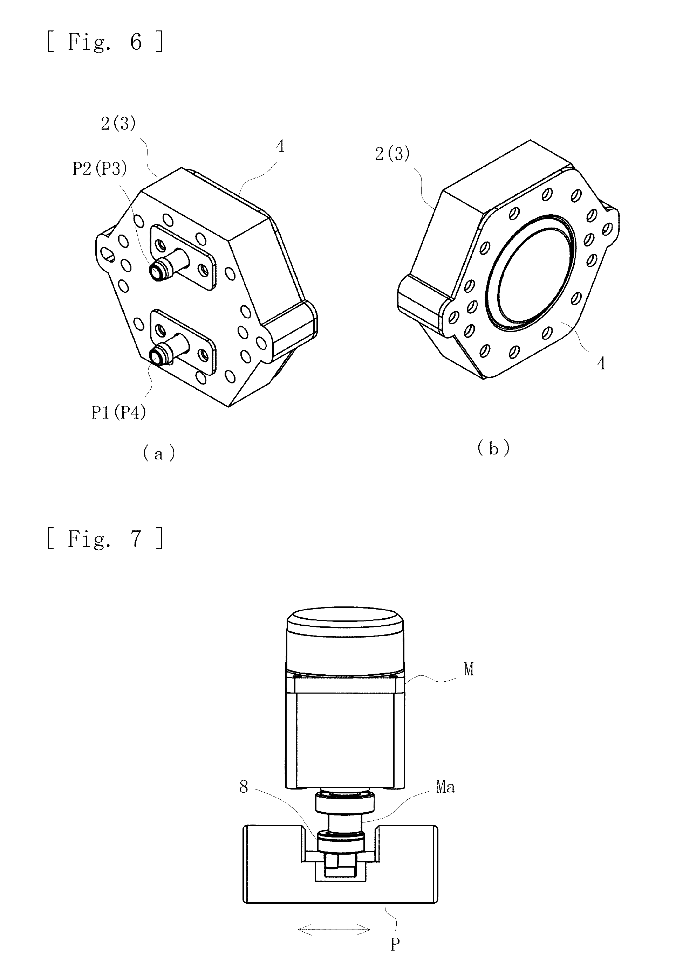

Specifically, the supply chamber 2 and the drain chamber 3 are provided with the respective dividing membranes 4 such that the supply-side accommodation part S1 and the drain-side accommodation part S2 are closed (in a fluid-tight manner). Therefore, in the supply chamber 2 and the drain chamber 3 detached from the pump body 7, as illustrated in Fig. 6, the supply-side accommodation part S1 and the drain-side accommodation part S2 are kept closed by the respective dividing membranes 4.

The supply chamber 2 or the drain chamber 3 is detachable from and attachable to the pump body 7 with the supply-side accommodation part S1 or the drain-side accommodation part S2 being closed by the corresponding dividing membrane 4. The pump body 7 includes a piston P (driven part), which is configured to move with the activation of the motor M (drive source) and in such a manner as to displace the dividing membranes 4. Specifically, the pump body 7 includes a pair of securing parts 7a, which is located at respective positions that adjoin the supply chamber 2 and the drain chamber 3; and a covering part 7b, which is located between the pair of securing parts 7a. The piston P is accommodated while being allowed to move along the pair of securing parts 8a and the covering part 7b.

The securing parts 7a of the pump body 7 are provided with respective moving membranes 5, which are fitted to the respective dividing membranes 4. Between each of the moving membranes 5 and a corresponding one of the ends of the piston P (driven part) is provided a filled part (S3 or S4), which has a predetermined capacity and is filled with incompressible liquid. As with the dividing membranes 4, the moving membranes 5 are each a flexible member (for example, a diaphragm made of a rubber material, a resin material, or the like) and are displaceable by bending with the transmission of the movement of the piston P through the incompressible liquid filling the filled parts (S3 and S4) .

The moving membranes 5 are attached to the securing part 7a for the supply chamber 2 and the securing part 7a for the drain chamber 3 such that the filled part S3 and the filled part S4 are closed (in a fluid-tight manner). Therefore, in the supply chamber 2 and the drain chamber 3 detached from the pump body 7, the filled parts (S3 and S4) are kept closed by the respective moving membranes 5. The filled parts (S3 and S4) are connected to respective filling ports P6, through which the incompressible liquid is allowed to be introduced and discharged.

Frame-shaped intermediate members 6 are provided between the dividing membrane 4 attached to the supply chamber 2 and the corresponding moving membrane 5 attached to the securing part 7a and between the dividing membrane 4 attached to the drain chamber 3 and the corresponding moving membrane 5 attached to the securing part 7a. The intermediate members 6 are each in contact with the dividing membrane 4 at one side thereof and is in contact with the moving membrane 5 at the other side thereof. The intermediate members 6 are each provided with a negative-pressure port P5, through which air between the dividing membrane 4 and the moving membrane 5 is allowed to be discharged so that a negative pressure is generated.

Discharging the air between the dividing membrane 4 and the moving membrane 5 through the negative-pressure port P5 generates a negative pressure between the dividing membrane 4 and the moving membrane 5 and thus brings the dividing membrane 4 and the moving membrane 5 into close contact with each other. With the dividing membranes 4 and the corresponding moving membranes 5 fitted to each other in such a manner as to be in close contact with each other as described above, when the piston P is moved to displace the moving membranes 5, the dividing membranes 4 are displaced while accurately following the displacements of the moving membranes 5. Thus, the suction and discharge of the dialysate into and from the supply-side accommodation part S1 and the suction and discharge of the drain liquid into and from the drain-side accommodation part S2 are accurately performed.

In the present embodiment, as illustrated in Fig. 8, pressure sensors J (pressure detectors) are provided to detect the respective pressures generated between the individual dividing membranes 4 and the corresponding moving membranes 5. If the dividing membrane 4 or the moving membrane 5 is damaged, the pressure between the dividing membrane 4 and the moving membrane 5 increases. Such damage to the dividing membrane 4 or the moving membrane 5 is detectable by the corresponding pressure sensor J. Specifically, if either of the dividing membrane 4 and the moving membrane 5 is damaged, the damaged part allows the liquid to flow in, which changes the negative pressure to a positive pressure. The pressure sensor J is capable of detecting such a pressure change and thus detecting the damage.

As illustrated in Fig. 7, the motor M as a drive source includes an output shaft Ma, to which a cam part 8 is attached. The motor M is capable of moving the piston P with the aid of the cam part 8. Specifically, when the motor M is activated and the output shaft Ma rotates, the cam part 8 causes the piston P to move to the left and right in the drawing, whereby the moving membranes 5 and the dividing membranes 4 for the supply chamber 2 and the drain chamber 3 are displaced.

The supply chamber 2 or the drain chamber 3, the corresponding dividing membrane 4, the corresponding intermediate member 6, and the corresponding moving membrane 5 are coupled to one another with fastening means, such as a bolt and not illustrated, extending therethrough to the corresponding securing part 7a. At times of maintenance work or part replacement, the coupling with the fastening means is undone, so that the supply chamber 2 or the drain chamber 3 to which the dividing membrane 4 is attached and the intermediate member 6 are allowed to be detached from the securing part 7a to which the moving membrane 5 is attached.

In the present embodiment, when the supply chamber 2 and the drain chamber 3 are detached from the pump body 7 for the maintenance work or part replacement of the pump body 1, since the supply-side accommodation part S1 and the drain-side accommodation part S2 are closed by the respective dividing membranes 4 as illustrated in Fig. 6, dialysate or drain liquid remaining after the dialysis treatment is kept confined in the supply-side accommodation part S1 or the drain-side accommodation part S2.

Now, a blood purification apparatus to which the pump device 1 according to the present embodiment is applied will be described with reference to Fig. 8.

The dialysate introduction lines (L1a and L1b) and the dialysate drain lines (L2a and L2b) are provided with respective check valves V, whereby the flow direction of the dialysate is restricted. Specifically, the check valve V provided to the dialysate introduction line L1a allows the flow of the dialysate in the dialysate introduction line L1a from the supply source toward the pump device 1 while restricting the reverse flow. The check valve V provided to the dialysate introduction line L1b allows the flow of the dialysate in the dialysate introduction line L1b from the pump device 1 toward the blood purifier H while restricting the reverse flow.

The check valve V provided to the dialysate drain line L2a allows the flow of the dialysate in the dialysate drain line L2a from the pump device 1 toward the dialysate draining means while restricting the reverse flow. The check valve V provided to the dialysate drain line L2b allows the flow of the dialysate in the dialysate drain line L2b from the blood purifier H toward the pump device 1 while restricting the reverse flow.

The blood purifier H applied to the present embodiment has a blood introduction port a, which is connectable to an arterial blood circuit Na through which a patient's blood is made to extracorporeally circulate; a blood delivery port b, which is connectable to a venous blood circuit Nb; the dialysate introduction port c, which is connectable to the dialysate introduction line L1b; and the dialysate drain port d, which is connectable to the dialysate drain line L2b. The blood purifier H includes inside the housing thereof a plurality of hollow fiber membranes (blood purification membranes).

The hollow fiber membranes included in the blood purifier H each have a plurality of microscopic holes. Therefore, when dialysate is made to flow outside the hollow fibers while blood is made to flow inside the hollow fibers, unnecessary matter (waste products) in the blood are allowed to permeate through the hollow fiber membranes to the dialysate and are thus removed. Between the dialysate drain line L2a and the dialysate drain line L2b is connected a bypass line L3, which is provided with an ultrafiltration pump Q. Activating the ultrafiltration pump Q enables ultrafiltration, in which water is removed from the patient's blood flowing through the blood purifier H.

Activating the motor M of the pump device 1 enables the introduction of the dialysate into the blood purifier H and the discharge of the drain liquid from the blood purifier H. Specifically, during the process in which the motor M of the pump device 1 is activated to cause the piston P to undergo a reciprocating motion inside the pump body 7, when the piston P moves toward the supply chamber 2, the dividing membranes 4 and the moving membranes 5 are displaced in such a manner as to reduce the capacity of the supply-side accommodation part S1 while increasing the capacity of the drain-side accommodation part S2. Accordingly, the dialysate is discharged from the liquid-supply-side connection port P2 and is thus introduced into the blood purifier H, whereas the drain liquid is suctioned into the liquid-drain-side connection port P3 and is thus discharged from the blood purifier H.

On the other hand, during the process in which the motor M of the pump device 1 is activated to cause the piston P to undergo a reciprocating motion inside the pump body 7, when the piston P moves toward the drain chamber 3, the dividing membranes 4 and the moving membranes 5 are displaced in such a manner as to increase the capacity of the supply-side accommodation part S1 while reducing the capacity of the drain-side accommodation part S2. Accordingly, the dialysate is suctioned into the supply-side accommodation part S1 from the liquid-supply-side connection port P1, whereas the drain liquid is discharged from the liquid-drain-side connection port P4 and is thus discharged from the drain-side accommodation part S2.

In the present embodiment, the flows of the dialysate and the drain liquid are settable in desired directions with the use of the check valves V. Therefore, the dialysate introduction lines (L1a and L1b) and the dialysate drain lines (L2a and L2b) do not need to be provided with additional pumps or electromagnetic valves. While the present embodiment employs a configuration in which the check valves V are connected to the dialysate introduction lines (L1a and L1b) and the dialysate drain lines (L2a and L2b), such a check valve may be attached to the supply chamber 2 or the drain chamber 3 of the pump device 1.

According to the present embodiment, the dividing membranes 4 are each a flexible member attached to a corresponding one of the supply chamber 2 and the drain chamber 3 in such a manner as to close a corresponding one of the supply-side accommodation part S1 and the drain-side accommodation part S2. Furthermore, the supply chamber 2 or the drain chamber 3 is detachable from and attachable to the pump body 7 with the supply-side accommodation part S1 or the drain-side accommodation part S2 being closed by the corresponding dividing membrane 4. Therefore, remaining dialysate or drain liquid is prevented from scattering to the outside when the chamber (supply chamber 2 or the drain chamber 3) is detached from the pump body 7.

The pump body 7 according to the present embodiment is provided with the moving membranes 5 fitted to the respective dividing membranes 4, and the space between each of the moving membranes 5 and the piston P (driven part) is filled with incompressible liquid. Therefore, the movement of the piston P is transmitted through the incompressible liquid to the dividing membranes 4 and the moving membranes 5 and thus displaces the dividing membranes 4 and the moving membranes 5. Furthermore, the incompressible liquid is sealed in by the moving membranes 5 and is therefore prevented from scattering to the outside when the chamber (supply chamber 2 or drain chamber 3) is detached from the pump body 7.

The negative-pressure ports P5 are each provided to generate a negative pressure between a corresponding one of the dividing membranes 4 and a corresponding one of the moving membranes 5 in such a manner as to bring the dividing membrane 4 and the moving membrane 5 into close contact with each other. Therefore, when the chamber (the supply chamber 2 or drain chamber 3) is attached to the pump body 7, the dividing membrane 4 and the moving membrane 5 are assuredly and easily brought into close contact with each other to be fitted to each other. Accordingly, the dividing membrane 4 accurately follows the moving membrane 5 while the piston P is moving. The pressure sensors J (pressure detectors) are each provided to detect the pressure generated between a corresponding one of the dividing membranes 4 and a corresponding one of the moving membranes 5. If the dividing membrane 4 or the moving membrane 5 is damaged, the pressure between the dividing membrane 4 and the moving membrane 5 increases. Such damage to the dividing membrane 4 or the moving membrane 5 is detectable by the pressure sensor J. The filling ports P6 are each provided to allow the incompressible liquid to be introduced into and discharged from the space between a corresponding one of the moving membranes 5 and the piston P (driven part). Therefore, refilling of the incompressible liquid is easy.

The driven part is the piston P configured to undergo a reciprocating motion inside the pump body 7. The supply chamber 2 and the drain chamber 3 are located at respective positions corresponding to the two respective ends of the piston P. Therefore, the reciprocating motion of the piston P is efficiently transmitted to the dividing membranes 4 for displacement. Accordingly, the suction and discharge of the dialysate and the suction and discharge of the drain liquid are smoothly performed.

Now, a pump device according to another embodiment of the present invention will be described.

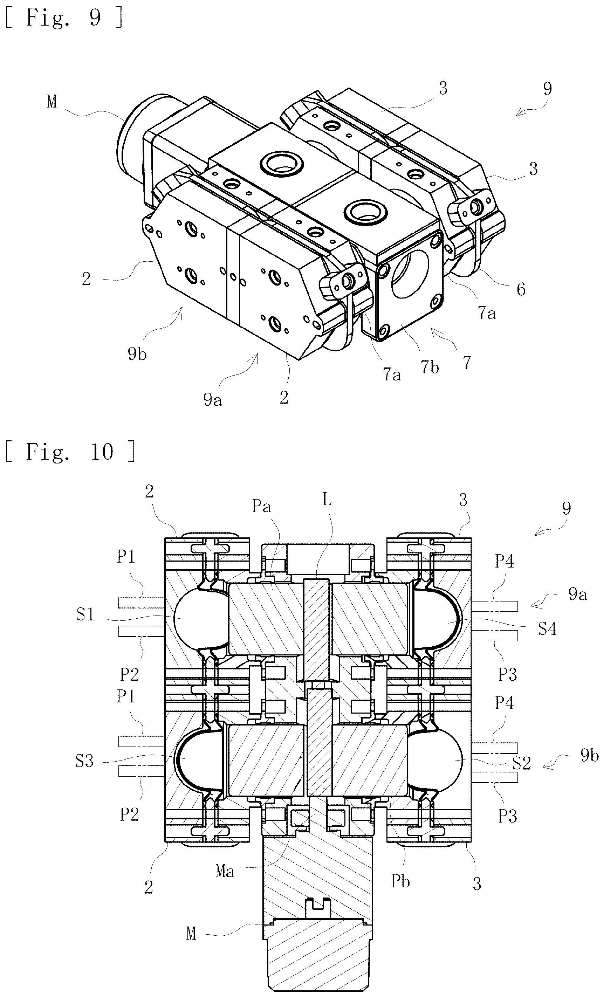

As illustrated in Figs. 9 and 10, a pump device 9 according to the present embodiment includes a plurality (two in the present embodiment) of pistons Pa and Pb inside a pump body 7. A supply chamber 2 and a drain chamber 3 are provided at respective positions corresponding to the two respective ends of each of the pistons Pa and Pb. Components that are the same as or similar to those of the above embodiment are denoted by the same reference signs, and detailed descriptions of such components are omitted.

The pump device 9 according to the present embodiment further includes a motor M, which serves as a drive source. The motor M has an output shaft Ma, to which a crankshaft L is attached astride the plurality of pistons Pa and Pb. Activating the motor M causes, through the crankshaft L, the pistons Pa and Pb to undergo respective reciprocating motions that are in opposite phases (the pistons Pa and Pb move in opposite directions), whereby the introduction of the dialysate into the blood purifier H and the discharge of the drain liquid from the blood purifier H are performed.

Specifically, the pump device 9 according to the present embodiment includes a first pump unit 9a, which includes the piston Pa serving as a driven part; and a second pump unit 9b, which includes the piston Pb serving as a driven part. Activating a single drive source (the motor M) causes, through the crankshaft L, the pistons Pa and Pb of the first pump unit 9a and the second pump unit 9b to undergo respective reciprocating motions that are in opposite phases.

During the process in which the motor M of the pump device 9 is activated to cause the pistons Pa and Pb to undergo the reciprocating motions in opposite phases inside the pump body 7, in the first pump unit 9a, when the piston Pa moves toward the supply chamber 2, the dividing membranes 4 and the moving membranes 5 are displaced in such a manner as to reduce the capacity of the supply-side accommodation part S1 while increasing the capacity of the drain-side accommodation part S2. Meanwhile, in the second pump unit 9b, the piston Pb moves toward the drain chamber 3 and displaces the dividing membranes 4 and the moving membranes 5 in such a manner as to increase the capacity of the supply-side accommodation part S1 while reducing the capacity of the drain-side accommodation part S2.

During the process in which the motor M of the pump device 9 is activated to cause the pistons Pa and Pb to undergo the reciprocating motions in opposite phases inside the pump body 7, in the first pump unit 9a, when the piston Pa moves toward the drain chamber 3, the dividing membranes 4 and the moving membranes 5 are displaced in such a manner as to increase the capacity of the supply-side accommodation part S1 while reducing the capacity of the drain-side accommodation part S2. Meanwhile, in the second pump unit 9b, the piston Pb moves toward the supply chamber 2 and displaces the dividing membranes 4 and the moving membranes 5 in such a manner as to reduce the capacity of the supply-side accommodation part S1 while increasing the capacity of the drain-side accommodation part S2.

That is, while the first pump unit 9a is suctioning the dialysate, the second pump unit 9b is caused to discharge the dialysate. Furthermore, while the first pump unit 9a is discharging the dialysate, the second pump unit 9b is caused to suction the dialysate. Thus, continuous introduction of the dialysate into the blood purifier H is enabled. On the other hand, while the first pump unit 9a is suctioning the drain liquid, the second pump unit 9b is caused to discharge the drain liquid. Furthermore, while the first pump unit 9a is discharging the drain liquid, the second pump unit 9b is caused to suction the drain liquid. Thus, continuous discharge of the drain liquid from the blood purifier H is enabled.

According to the present embodiment, the plurality of pistons Pa and Pb is provided inside the pump body 7. Furthermore, the supply chamber 2 and the drain chamber 3 are provided at respective positions corresponding to the two respective ends of each of the pistons Pa and Pb. Furthermore, activating the motor M (drive source) causes the plurality of pistons Pa and Pb to undergo respective reciprocating motions that are in opposite phases. Therefore, continuous introduction of the dialysate into the blood purifier H and continuous discharge of the drain liquid from the blood purifier H are enabled, which suppresses pulsations.

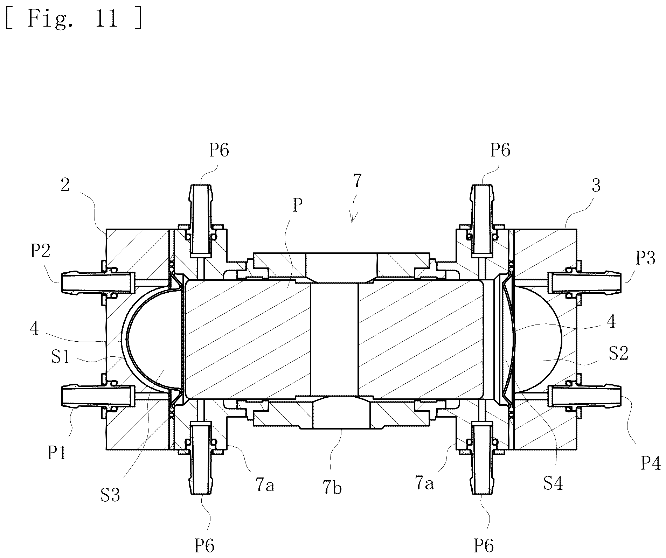

While the present embodiments have been described above, the present invention is not limited thereto. For example, as in a pump device illustrated in Fig. 11, while the supply chamber 2 and the drain chamber 3 are provided with respective dividing membranes 4, the securing parts 7a of the pump body 7 may be provided with no moving membranes 5. Alternatively, in the pump device including the dividing membranes 4 and the moving membranes 5, the negative-pressure ports P5 may be omitted (the dividing membranes 4 and the moving membranes 5 may not necessarily be brought into close contact with each other). As an alternative to the pump device in which the dividing membranes 4 and the moving membranes 5 are brought into close contact with each other under a negative pressure generated between the two, for example, the dividing membranes 4 and the moving membranes 5 may be bonded to each other with an adhesive material, or may be brought into close contact with each other with a magnetic force.

According to a first embodiment of the present invention, there is provided a pump device 1 including a supply chamber 2 to which a liquid-supply-side connection port (P1, P2) is connected and that has a supply-side accommodation part S1 configured to accommodate a predetermined volume of dialysate; a drain chamber 3 to which a liquid-drain-side connection port (P3, P4) is connected and that has a drain-side accommodation part S2 configured to accommodate a predetermined volume of drain liquid; dividing membranes 4 each being a flexible member attached to a corresponding one of the supply chamber 2 and the drain chamber 3 in such a manner as to close a corresponding one of the supply-side accommodation part S1 and the drain-side accommodation part S2, the dividing membranes 4 being displaceable relative to the supply-side accommodation part S1 and the drain-side accommodation part S2 in such a manner as to cause the dialysate to be suctioned into and discharged from the supply-side accommodation part S1 through the liquid-supply-side connection port (P1, P2) and cause the drain liquid to be suctioned into and discharged from the drain-side accommodation part S2 through the liquid-drain-side connection port (P3, P4); and a pump body 7 from and to which the supply chamber 2 or the drain chamber 3 is detachable and attachable with the supply-side accommodation part S1 or the drain-side accommodation part S2 being closed by the corresponding dividing membrane 4, the pump body 7 including a piston P (driven part) configured to move with activation of a motor M (drive source) and in such a manner as to displace the dividing membranes 4. Therefore, remaining dialysate or drain liquid is prevented from scattering to the outside when the chamber (supply chamber 2 or the drain chamber 3) is detached from the pump body 7.

According to a second embodiment of the present invention, in the pump device 1 according to the first embodiment, the pump body 7 is provided with moving membranes 5 fitted to the respective dividing membranes 4. Furthermore, a space between each of the moving membranes 5 and the piston P (driven part) is filled with incompressible liquid. Therefore, the movement of the piston P is transmitted through the incompressible liquid to the dividing membranes 4 and the moving membranes 5 and thus displaces the dividing membranes 4 and the moving membranes 5. Furthermore, the incompressible liquid is sealed in by the moving membranes 5 and is therefore prevented from scattering to the outside when the chamber (supply chamber 2 or drain chamber 3) is detached from the pump body 7.

According to a third embodiment of the present invention, the pump device 1 according to the second embodiment further includes a negative-pressure port P5 through which a negative pressure is generated between each of the dividing membranes 4 and a corresponding one of the moving membranes 5 in such a manner as to bring the dividing membrane 4 and the moving membrane 5 into close contact with each other. Therefore, when the chamber (supply chamber 2 or drain chamber 3) is attached to the pump body 7, the dividing membrane 4 and the moving membrane 5 are assuredly and easily brought into close contact with each other to be fitted to each other. Accordingly, the dividing membrane 4 accurately follows the moving membrane 5 while the piston P is moving.

According to a fourth embodiment of the present invention, the pump device 1 according to the second embodiment includes a pressure sensor J that detects a pressure generated between each of the dividing membranes 4 and a corresponding one of the moving membranes 5. If the dividing membrane 4 or the moving membrane 5 is damaged, the pressure between the dividing membrane 4 and the moving membrane 5 increases. Such damage to the dividing membrane 4 and the moving membrane 5 is detectable by the pressure sensor J.

According to a fifth embodiment of the present invention, the pump device 1 according to the second embodiment further includes a filling port P6 through which the incompressible liquid is introduced into and discharged from the space between each of the moving membranes 5 and the piston P (driven part). Therefore, refilling of the incompressible liquid is easy.

According to a sixth embodiment of the present invention, in the pump device 1 according to the second embodiment, the driven part is a piston P (Pa, Pb) configured to undergo a reciprocating motion inside the pump body 7. Furthermore, the supply chamber 2 and the drain chamber 3 are located at respective positions corresponding to two respective ends of the piston P (Pa, Pb). Therefore, the reciprocating motion of the piston P is efficiently transmitted to the dividing membranes 4 for displacement. Accordingly, the suction and discharge of the dialysate and the suction and discharge of the drain liquid are smoothly performed.

According to a seventh embodiment of the present invention, in the pump device 1 according to the sixth embodiment, a plurality of pistons Pa and Pb is provided inside the pump body 7. Furthermore, the supply chamber 2 and the drain chamber 3 are provided at respective positions corresponding to two respective ends of each of the pistons Pa and Pb. Furthermore, activating the motor M (drive source) causes the plurality of pistons Pa and Pb to undergo respective reciprocating motions that are in opposite phases. Therefore, continuous introduction of the dialysate into the blood purifier H and continuous discharge of the drain liquid from the blood purifier H are enabled, which suppresses pulsations.

### Industrial Applicability

The present invention is also applicable to any pump device having a different appearance, additional functions, or the like within the spirit of the present invention.

### Reference Signs List

1, 9 pump device
2 supply chamber
3 drain chamber
4 dividing membrane
5 moving membrane
6 intermediate member
7 pump body
7a securing part
7b covering part
8 cam part
9 pump device
9a first pump unit
9b second pump unit
M motor (drive source)
Ma output shaft
P piston (driven part)
P1, P2 liquid-supply-side connection port
P3, P4 liquid-drain-side connection port
P5 negative-pressure port
P6 filling port
S1 supply-side accommodation part
S2 drain-side accommodation part
S3, S4 filled part
J pressure sensor (pressure detector)

## Claims

1. A pump device comprising:
a supply chamber to which a liquid-supply-side connection port is connected and that has a supply-side accommodation part configured to accommodate a predetermined volume of dialysate;
a drain chamber to which a liquid-drain-side connection port is connected and that has a drain-side accommodation part configured to accommodate a predetermined volume of drain liquid;
dividing membranes each being a flexible member attached to a corresponding one of the supply chamber and the drain chamber in such a manner as to close a corresponding one of the supply-side accommodation part and the drain-side accommodation part, the dividing membranes being displaceable relative to the supply-side accommodation part and the drain-side accommodation part in such a manner as to cause the dialysate to be suctioned into and discharged from the supply-side accommodation part through the liquid-supply-side connection port and cause the drain liquid to be suctioned into and discharged from the drain-side accommodation part through the liquid-drain-side connection port; and
a pump body from and to which the supply chamber or the drain chamber is detachable and attachable with the supply-side accommodation part or the drain-side accommodation part being closed by the corresponding dividing membrane, the pump body including a driven part configured to move with activation of a drive source and in such a manner as to displace the dividing membranes.

2. The pump device according to claim 1, wherein the pump body is provided with moving membranes fitted to the respective dividing membranes, and a space between each of the moving membranes and the driven part is filled with incompressible liquid.

3. The pump device according to claim 2, further comprising a negative-pressure port through which a negative pressure is generated between each of the dividing membranes and a corresponding one of the moving membranes in such a manner as to bring the dividing membrane and the moving membrane into close contact with each other.

4. The pump device according to claim 2, further comprising a pressure detector configured to detect a pressure generated between each of the dividing membranes and a corresponding one of the moving membranes.

5. The pump device according to claim 2, further comprising a filling port through which the incompressible liquid is introduced into and discharged from the space between each of the moving membranes and the driven part.

6. The pump device according to claim 2, wherein the driven part is a piston configured to undergo a reciprocating motion inside the pump body, and the supply chamber and the drain chamber are located at respective positions corresponding to two respective ends of the piston.

7. The pump device according to claim 6, wherein the piston includes a plurality of pistons provided inside the pump body, the supply chamber and the drain chamber are provided at respective positions corresponding to two respective ends of each of the pistons, and activating the drive source causes the plurality of pistons to undergo respective reciprocating motions that are in opposite phases.
